Europäisches Patentamt

European Patent Office

Office européen des brevets

(11) Publication number: **0 171 158**
**B1**

(12) **EUROPEAN PATENT SPECIFICATION**

(45) Date of publication of patent specification: **01.02.89**

(51) Int. Cl.⁴: **C 12 Q 1/04, C 12 Q 1/26**

(21) Application number: **85304486.5**

(22) Date of filing: **24.06.85**

(54) Processes and materials for carrying out microchemical and microbiological tests.

(30) Priority: **22.06.84 GB 8416045**

(43) Date of publication of application:
**12.02.86 Bulletin 86/07**

(45) Publication of the grant of the patent:
**01.02.89 Bulletin 89/05**

(84) Designated Contracting States:
**AT BE CH DE FR GB IT LI NL SE**

(56) References cited:
**WO-A-84/03303**
**FR-A-2 196 386**
**US-A-4 245 043**
**US-A-4 345 027**
**US-A-4 495 293**

**CHEMICAL ABSTRACTS, vol. 82, no. 26, 30th June 1975, page 72, no. 172407x, Columbus, Ohio, US; J.R. ZDYSIEWICZ: "Interactions of 7-diethylamino-4-methylcoumarin with wool and indole", & AUST. J. CHEM. 1975, 28(4), 871-9**

(73) Proprietor: **RADIOMETER CORPORATE DEVELOPMENT LIMITED**
**The Manor Manor Royal**
**Crawley, West Sussex RH10 2PY (GB)**

(72) Inventor: **Beggs, Thomas Stewart**
**70 Somerford Road**
**Wellingborough Northants NN8 3EZ (GB)**
Inventor: **Sands, Thomas John**
**52B Somerford Road**
**Wellingborough Northants NN8 3EZ (GB)**

(74) Representative: **Jones, Helen M.M. et al**
**Gill Jennings & Every 53-64 Chancery Lane**
**London WC2A 1HN (GB)**

## Description

This invention relates to processes and materials for carrying out microchemical and microbiological tests. In particular, it relates to chemical tests to detect the presence of small quantities of the enzyme activity tryptophan deaminase, and/or the product of its activity on tryptophan, i.e. indolepyruvic acid, and the application of such tests to detecting the production of this enzyme activity by microorganisms in culture, i.e. to identifying tryptophan-deaminase-positive microorganisms.

A well established microbiological tryptophan deaminase test, for instance as described in FR—A—2196386, relies on the addition of acid ferric chloride solution to a microorganism culture grown on solid medium containing tryptophan for 18—24 hours, and observing intense red-brown coloration due to a ferric complex with indolepyruvic acid if tryptophan deaminase activity is present.

As it stands, this test generally requires visual assessment of colour development, and it involves the use and handling of the somewhat noxious and inconvenient acid ferric chloride.

US—A—4345027 discloses a method for assaying a mammalian cell culture for mutagenesis in which the fixed cell culture is stained with a histochemical stain, which stains cellular material, for instance an enzyme, present in normal cells only, and then with a fluorescent stain which stains normal and abnormal cells. The stains are chosen so that the histochemical stain absorbs the wavelengths that the fluorescent stain emits. The degree of mutagenesis is assessed by observing the fluorescence from the abnormal cells.

The aim of this invention is to provide a tryptophan deaminase test that can be assessed by fluorescence in the resulting test material. It is also the aim of this invention to provide tests that can give a fluorescent result in a form suited to automatic fluorimetric assessment, especially for example by fluorimetry that uses similar excitation and emission wavelengths as other and possibly hitherto chemically unrelated microbial tests, which can therefore be assessed simultaneously or in the same batch by similar or the same instrumentation. A further aim of the invention is to provide tryptophan deaminase detection tests adapted to microbiological use for assessment of the tryptophan deaminase activity of microbial cultures, e.g. to assist in their identification, in which the use of noxious reagents to develop a colour result is minimised or avoided.

According to the present invention, a microorganism is cultured in the presence of enough L-tryptophan to allow formation of indolepyruvic acid if possible, and the fluorescence-quenching effect of the culture medium, after its exposure to reaction with ferric ions (or other chromogen-forming metal ions, especially other transition-metal ions), upon a fluorophor such as a fluorescent coumarin derivative is assessed fluoroscopi- cally or fluorimetrically. TDA-positive organisms then give substantial quenching of the coumarin fluorescence.

The invention correspondingly provides a microbiological culture test process for detecting the presence of tryptophan-deaminase-positive microorganisms, which comprises

(a) culturing a microorganism to be tested in the presence of L-tryptophan in an effective amount and for a period sufficient to allow production of indolepyruvic acid by the organism if it is of tryptophan-deaminase-positive character,

(b) exposing the culture to a chromogenic amount of metal ions which are chromogenic with indolepyruvate, and to a fluorophor, the ions and fluorophor being present throughout the culture period, and

(c) assessing the fluorescence of the culture after steps (a) and (b), thereby to show a reduction in fluorescence in the presence of a tryptophan-deaminase-positive microorganism.

Preferred coumarin derivatives are 4-methyl-umbelliferone, and 7-amino-4-methyl coumarin, more preferably the latter.

The quantity of L-tryptophan present is generally more than the traces usually present in for example yeast extracts alone. Amounts of L-tryptophan of more than about 0.1%, e.g. 0.5%, and up to about 2%, are useful for example. The known TDA tests also employ such larger amounts than usual of L-tryptophan.

We find advantageously that with this system it is not necessary to use strongly acid ferric chloride. A chromogenic amount of the metal ions is that which is effective to generate a fluorescence-quenching quantity of coloured product in the presence of indolepyruvate. We include the metal ions at low but effective concentration in the culture medium itself, in the form of a ferric chloride or ferric nitrate or ferric citrate ingredient at for example above about 1 millimolar and up to about 15 millimolar concentration. Most preferred is ferric citrate of the order of about 3 millimolar. As the fluorophor is also included in the culture medium it can be seen that this tryptophan deaminase test avoids the need for secondary reagents altogether, which simplifies manipulation to a very useful extent.

Example:

An example of a suitable culture broth formulation to carry out the test is as follows:—

| | |
|---|---|
| Yeast extract | 0.10% |
| Sodium chloride | 0.35% |
| $NaNH_4HPO_4 . 4H_2O$ | 0.20% |
| $K_2HPO_4 . 3H_2O$ | 0.20% |
| L-Tryptophan | 0.50% |
| Ferric citrate | 0.072% |
| 7-Methyl-4-amino-coumarin | 10 micro-molar |

A microorganism can be most suitably inoculated into this liquid medium at an inoculum level of about $10^7$ cells/ml, incubated for preferably about 4—6 hours, and the fluorescence measured by excitation at about 363 nm and detection at about 450 nm, e.g. using a Perkin-Elmer 1000 commercially available fluorometer. It is found that marked quenching of fluorecence results from the growth of at least the vast majority of tryptophan-deaminase-positive microorganisms but not from the growth of at least the vast majority of tryptophan-deaminase-negative organisms.

It can be seen from the above description that the invention provides a method of tryptophan deaminase activity detection or estimation in microorganisms: the invention also comprises materials for carrying out such tests. In particular, the material can comprise a microbiological broth containing tryptophan, and a source of ferric or other chromogen-forming metal ions, as well as a source of fluorophor, optionally and preferably all combined together, in suitable quantities (e.g. those given in detail above). If desired, a convenient embodiment can be used in which the indicated broth constituents, for a selection of them including the essential tryptophan, and optionally also the ion source and/or the fluorophor), can be presented in dry form in one or more wells of a prepared (usually sterile) microtitre tray for use in carrying out microbial culture tests (usually protected by a removable adhesive sealing cover). An alternative dry form presents some or all of the materials in or associated with (usually sterile) containers for preparing a microbial inoculum suspension, e.g. a bottle or other container with a dry preparation of the materials contained therein; or a closure for such a bottle, such as a screw cap, with a dry preparation of the materials carried on a surface which in use is to contact the inoculum so that the materials may be dispersed therethrough, such as the inner surface of the screw cap or the surface of a wad placed within the screw cap; or in a dry form on a carrier material suitable to be added to such an inoculum or to a culture well, so that the materials can be dispersed through the inoculum or culture suspension, such as strips or discs of paper or other cellulosic or non-cellulosic, fibrous or non-fibrous carrier sheet material.

All such preparations are desirably maintained dry and sterile before use, e.g. sealed within sterile foil sealing or packaging material.

One of the special advantages of the processes described herein is that the tryptophan deaminase test gives a fluoroscopic/fluorometric result that can be measured optically with the same excitation/emission wavelengths as a number of other (known in themselves) microbial culture tests (e.g. in the presence of various quantities or types of antimicrobial) giving a variably fluorescent result: e.g. culture tests in which a fluorogenic substrate such as 4-methyl umbelliferone phosphate or other ester and/or an aminoacyl or peptidyl 7-amino-4-methyl coumarin is hydrolysed to fluorophor during growth of the microorganism if this growth occurs.

Accordingly, combination tests including cultures for tryptophan deaminase testing and such antimicrobial tests are also included within the scope of the invention.

Other modifications and variations within the scope of the described invention will be apparent to the skilled reader as a result of reading the above disclosure, and the several features described by way of example can be presented in any desired combinations.

## Claims

1. A microbiological culture test process for detecting the presence of tryptophan-deaminase-positive microorganisms, which comprises

(a) culturing a microorganism to be tested in the presence of L-tryptophan in an effective amount and for a period sufficient to allow production of indolepyruvic acid by the organism if it is of tryptophan-deaminase-positive character,

(b) exposing the culture to a chromogenic amount of metal ions which are chromogenic with indolepyruvate, and to a fluorophor whose fluorescence is capable of being quenched by the indolepyruvate-metal ion complex, the ions and fluorophor being present throughout the culture period, and

(c) assessing the fluorescence of the culture after steps (a) and (b), thereby to show a reduction in fluorescence in the presence of a tryptophan-deaminase-positive microorganism.

2. A process according to claim 1 in which the fluorophor is a fluorescent coumarin derivative.

3. A process according to claim 2 in which the fluorophor is 7-amino-4-methyl coumarin or 4-methylumbelliferone.

4. A process according to any preceding claim in which the chromogenic metal ions are ferric ions.

5. A process according to any preceding claim, in which the metal ion concentration is up to about 15 millimolar.

6. A microbial culture test medium suitable for carrying out a culture test process according to claim 1, comprising besides usual microbial culture medium ingredients, L-tryptophan, a fluorophor, and a chromogenic metal ion capable of forming with indolepyruvate a coloured product that quenches the fluoroescence of the fluorophor.

7. A microbial culture test medium according to claim 6, wherein said fluorophor is a fluorescent coumarin, and the chromogenic metal ions are ferric ions.

8. A microbial culture test medium according to claim 6 or 7, wherein the concentration of L-tryptophan is of the order of 0.5% w/v, and the concentration of the metal ions is up to about 15 millimolar.

9. A test kit comprising prepared dry reagents for carrying out a microbial culture test according to claim 1, comprising a test tray prepared with a plurality of receptacles and/or a container for preparing microbial inoculum suspension, containing in dry form in one or more receptacles or containers thereof reagents for carrying out said culture test, said reagents comprising L-tryptophan and a salt of a chromogenic metal ion which is chromogenic with indolepyruvate, and a fluorophor capable of being quenched by the chromogenic product of the metal ion and indolepyruvate.

10. A test kit according to claim 9 which comprises L-tryptophan, said salt and said fluorophor in the same container or receptacle.

11. A test kit according to claim 9 or 10 in which the salt is a ferric salt and the fluorophor is a fluorescent coumarin.

## Patentansprüche

1. Mikrobiologisches Kulturtestverfahren zur Feststellung der Anwesenheit von tryptophandeaminase-positiven Mikroorganismen, welches umfaßt

(a) das Kultivieren eines zu testenden Mikroorganismus in Anwesenheit von L-Tryptophan in einer wirksamen Menge und für eine ausreichende Dauer, um die Erzeugung von Indolbrenztraubensäure durch den Organismus zuzulassen, wenn dieser tryptophan-deaminase-positiv ist;

(b) das Behandeln der Kultur mit einer chromogenen Menge von Metallionen, die mit Indolpyruvat chromogen sind, und mit einem Fluorophor, dessen Fluoreszenz durch den Indolepyruvat-Metallionen-Komplex gedämpft werden kann, wobei die Ionen und das Fluorophor während der gesamten Kultivierungsperiode anwesend sind, und

(c) die Bestimmung der Fluoreszenz der Kultur nach den Stufen (a) und (b), um so eine Verminderung der Fluoreszenz in Anwesenheit eines tryptophan-deaminase-positiven Mikroorganismus nachzuweisen.

2. Verfahren nach Anspruch 1, bei welchem der Fluorophor ein fluoreszierendes Cumarinderivat ist.

3. Verfahren nach Anspruch 2, bei welchem der Fluorophor 7-Amino-4-methylcumarin oder 4-Methylumbelliferon ist.

4. Verfahren nach irgendeinem vorhergehenden Anspruch, bei welchem die chromogenen Metallionen Eisen-III-ionen sind.

5. Verfahren nach irgendeinem vorhergehenden Anspruch, bei welchem die Metallionenkonzentration bis zu etwa 15 mMol beträgt.

6. Mikrobielles Kulturtestmedium, geeignet zur Durchführung eines Kulturtestverfahrens nach Anspruch 1, das neben üblichen, mikrobiellen Kulturmedienbestandteilen L-Tryptophan, einen Fluorophor und ein chromogenes Metallion, das mit Indolpyruvat ein gefärbtes Produkt bilden kann, welches die Fluoreszenz des Fluorophors dämpft, umfaßt.

7. Mikrobielles Kulturtestmedium nach Anspruch 6, bei welchem der Fluorophor ein fluoreszierendes Cumarin ist und die chromogenen Metallionen Eisen-III-ionen sind.

8. Mikrobielles Kulturtestmedium nach Anspruch 6 oder 7, bei welchem die Konzentration von L-Tryptophan in der Größenordnung von 0,5% Gew./Vol. liegt und die Konzentration der Metallionen bis zu etwa 15 mMol beträgt.

9. Testausrüstung (Kit), die vorbereitete, trockene Reagenzien zur Durchführung eines mikrobiellen Kulturtests nach Anspruch 1 umfaßt und die eine Testplatte, versehen mit einer Vielzahl von Aufnahmelöchern und/oder einen Behälter zur Herstellung einer mikrobiellen Inoculumsuspension umfaßt, enthaltend in trockener Form in einem oder mehreren Aufnahmelöchern oder Behältern die Reagenzien zur Durchführung dieses Kulturtests, woebi diese Reagenzien L-Tryptophan und ein Salz eines chromogenen Metallions, das mit Indolpyruvat chromogen ist, und einen Fluorophor, der durch das chromogene Produkt aus Metallion und Indolpyruvat gedämpft werden kann, umfassen.

10. Testausrüstung (Kit) nach Anspruch 9, die das L-Tryptophan, das Salz und den Fluorophor im gleichen Behälter oder Aufnahmeloch umfaßt.

11. Testausrüstung (Kit) nach Anpruch 9 oder 10, bei welcher das Salz ein Eisen-II-salz ist und der Fluorophor ein fluoreszierendes Cumarin ist.

## Revendications

1. Un procédé de test de culture microbiologique pour déceler la présence des micro-organismes tryptophane-désaminase-positifs, qui comprend:

(a) la culture d'un micro-organisme à tester en présence du L-tryptophane en une quantité efficace et pendant une période de temps suffisante pour permettre la production de l'acide indole pyruvique par l'organisme lorsque ce dernier est de caractére tryptophane-désaminase-positif,

(b) l'exposition de la culture à une quantité chromogène d'ions métalliques qui sont chromogènes avec l'indole pyruvate, et à un fluorophore dont la fluorescence est susceptible d'être éteinte par le complexe indole pyruvate-ions métalliques, les ions et le fluorophore étant présents pendant toute la période de culture, et

(c) l'estimation de la fluorescence de la culture après les étapes (a) et (b), pour montrer une réduction de la fluorescence en présence d'un micro-organisme tryptophane-désaminase-positif.

2. Un procédé selon la revendication 1, selon laquelle le fluorophore est un dérivé de coumarine fluorescent.

3. Un procédé selon la revendication 2, selon lequel le fluorophore est la 7-amino-4-méthylcoumarine ou la 4-méthylombelliférone.

4. Un procédé selon l'une quelconque des revendications précédentes, selon lequel les ions métalliques chromogènes sont des ions ferriques.

5. Un procédé selon l'une quelconque des revendications précédentes, selon lequel la concentration en ions métalliques peut aller jusqu'à 15 mmol.

6. Un milieu de test de culture microbienne utile pour la mise en oeuvre du procédé de test de culture selon la revendication 1, comprenant outre les ingrédients de milieu de culture microbienne usuels, le L-tryptophane, un fluorophore, et un ion métallique chromogène capable de former avec l'indole pyruvate un produit coloré qui provoque l'extinction de la fluorescence du fluorophore.

7. Un milieu de test de culture microbienne selon la revendication 6, selon lequel ledit fluorophore est une coumarine fluorescente et les ions métalliques chromogènes sont des ions ferriques.

8. Un milieu de tests de culture microbienne selon la revendications 6 ou 7, selon lequel la concentration en L-tryptophane est de l'ordre de 0,5% en poids/volume et la concentration des ions métalliques peut atteindre 15 mmol environ.

9. Une trousse d'essai comprenant des réactifs secs préaprés pour la mise en oeuvre d'un test de culture microbienne selon la revendication 1, comprenant un plateau de test préparé avec plusieurs réceptacles et/ou un conteneur pour la préparation de la suspension d'inoculum microbien, contenant sous forme sèche dans un ou plusieurs réceptacles ou conteneurs des réactifs pour la mise en oeuvre dudit test de culture, lesdits réactifs comprenant le L-tryptophane et un sel d'un ion métallique chromogène qui est chromogène avec l'indole pyruvate, et un fluorophore susceptible d'extinction par le produit chromogène de l'ion métallique et de l'indole pyruvate.

10. Une trousse d'essai selon la revendication 9, qui comprend le L-tryptophane, ledit sel et ledit fluorophore dans le même conteneur ou réceptacle.

11. Une trousse d'essai selon la revendication 9 ou 10, selon laquelle le sel est en sel ferrique et le fluorophore est une coumarine fluorescente.